# EUROPEAN PATENT SPECIFICATION

(11) **EP 4 483 290 B1**
(45) Date of publication and mention of the grant of the patent: **14.10.2026**
(21) Application number: 22721141.4
(22) Date of filing: 25.02.2022
(51) Int. Cl.: G06K 19/07, A23N 15/06, G01N 33/02, G06K 19/077

(54) **RFID SYSTEM AND METHOD FOR MONITORING THE RIPENING DEGREE OF A PLURALITY OF FRUITS**
RFID-SYSTEM UND -VERFAHREN ZUR ÜBERWACHUNG DES REIFEGRADES MEHRERER FRÜCHTE
SYSTÈME RFID ET PROCÉDÉ DE SURVEILLANCE DU DEGRÉ DE MATURATION D'UNE PLURALITE DE FRUITS

(43) Date of publication of application: 01.01.2025
(73) Proprietor: ILIP S.r.l., 40053 Valsamoggia (BO) (IT)
(72) Inventor: GARAVAGLIA, Luigi, 40053 Valsamoggia (BO) (IT); BIGNAMI, Filippo, 40053 Valsamoggia (BO) (IT); MARROCCO, Gaetano, 00133 Roma (IT); OCCHIUZZI, Cecilia, 00133 Roma (IT); D'UVA, Nicola, 00133 Roma (IT); AMENDOLA, Sara, 00133 Roma (IT); CAMERA, Francesca, 00133 Roma (IT)
(74) Representative: Botti & Ferrari S.p.A.
(86) International application number: PCT/IT2022/000013
(87) International publication number: WO 2023/161967

(56) References cited:
- EP-A1- 3 648 009
- US-A1- 2007 273 507
- US-A1- 2016 026 911
- OCCHIUZZI CECILIA ET AL: "Automatic Monitoring of Fruit Ripening Rooms by UHF RFID Sensors and Machine Learning", 2021 IEEE INTERNATIONAL CONFERENCE ON RFID TECHNOLOGY AND APPLICATIONS (RFID-TA), IEEE, 6 October 2021 (2021-10-06), pages 187 - 190, XP034033957, DOI: 10.1109/RFID-TA53372.2021.9617278
- FORMISANO FABRIZIO ET AL: "Auxiliary smart gas sensor prototype plugged in a rfid active tag for ripening evaluation", 2015 XVIII AISEM ANNUAL CONFERENCE, IEEE, 3 February 2015 (2015-02-03), pages 1 - 4, XP032751410, DOI: 10.1109/AISEM.2015.7066800
- JOSEPH M. AZZARELLI ET AL: "Wireless gas detection with a smartphone via rf communication", PROCEEDINGS OF THE NATIONAL ACADEMY OF SCIENCES, vol. 111, no. 51, 8 December 2014 (2014-12-08), pages 18162 - 18166, XP055598674, ISSN: 0027-8424, DOI: 10.1073/pnas.1415403111

## Description

### Field of application

In its more general aspect, the present invention relates to the sector of fruit and vegetables.

In particular, the present invention relates to a system for monitoring the state or the degree of ripeness of a plurality of fruits, in particular but not exclusively tropical fruits, based on RFID technology. Said system comprises a support having a plurality of cavities for containing said fruits, at least one RFID tag operating in the UHF band, associated to each fruit and arranged in contact with or close to said fruit and at least one interrogation antenna operatively associated to at least one RFID tag, and means for processing electromagnetic signals (data) relative to or related to the degree of ripeness of said fruits coming from the interrogation of said RFID tags by means of said at least one interrogation antenna.

The present invention also relates to a method for monitoring a plurality of fruits that uses a system based on RFID technology as mentioned above.

The present invention further relates to a support suitable for the use in a system for monitoring the ripeness of a plurality of fruits as above, the support comprising a plurality of cavities for containing said fruits, one for each fruit, and at least one housing seat, preferably a plurality of housing seats, containing a respective interrogation antenna, each interrogation antenna being operatively associated to at least one RFID tag, preferably being associated to a group of RFID tags.

### Prior art

In the sector of fruit and vegetables, the increasing demand for fruits, such as tropical fruits, meeting higher standards of quality and freshness, results in the need of monitoring the state or the degree of ripeness of the fruits from harvest and possible artificial ripening in designated ripening cells to the full distribution chain.

Traditionally, the state of ripeness of fruits is established on the basis of some characteristics of the fruit, such as for example soluble solids, pulp hardness, etc., which can be determined with simple instrumentation such as a refractometer, a penetrometer, etc. Said techniques, however, have the drawback of a poor representativeness and usually entail the destruction of the analyzed fruits.

More recently, non-destructive systems for monitoring the state of ripeness of fruits, such as tropical fruits, have been introduced, including in particular systems based on RFID technology.

As it is known, a RFID device comprises one or more RFID tags, commonly called RFID tags or transponders, which are intended to be attached to the object to identify or detect, and interrogation means such as a reader having an antenna capable of reading radio-frequency (RF) electromagnetic data (signals) transmitted by the RFID tags.

In particular, in the field of fruit monitoring, the RFID tag may be arranged in contact with or close to a respective fruit (for example in a package containing them as in the case of the so-called "smart" packaging), receives electromagnetic signals via the antenna of the reader and returns a backscattered signal affected by variations related to the state of ripeness of the fruit associated thereto.

It is in fact known that fruits have peculiar dielectric characteristics that change over time in connection to changes the fruit undergoes due to ripening (moisture, acidity, sugars) or to physiological alterations (texture) that occur during its storage.

Therefore, the passive antenna which is part of the RFID tags in contact with or close to the respective fruits accordingly modifies its matching impedance, acting as a transducer which sends a signal reflecting said modification.

The reader detects the electromagnetic signals reflected by the RFID tags in a passive way, such as the echoes of a conventional radar, and it can process said signals through suitable classification algorithms so as to decode the state of the fruits and classify them.

A system for monitoring the degree of ripeness of fruits based on RFID technology as mentioned above is described for example in patent EP 3648009 in the name of the Applicant.

US 2016/0026911 deals with a standardized storage box having an array of storage slots, each slot being capable of receiving a cryogenic vial. A box lid is used to cover the storage box. The lid comprises an array of RFID tags, each tag being positioned on the box lid such that when the box lid is in use covering the storage box, the RFID tag is aligned with a storage slot in the box for a cryogenic vial. Each RFID tag therefore acts as an indication reference for the sample located in the slot with which it is aligned.

US 2007/0273507 discloses a sensor for monitoring and communicating data related to a perishable item, the sensor being adapted to operate with an RFID device including an antenna for receiving signals from an RF reader, the sensor comprising: a sensor module that monitors time and temperature of a perishable item, that determines a current freshness status based on the time and temperature, and that selectively transmits data representing the freshness status; a communication interface with the RFID device, the interface allowing an RFID reader to retrieve data representing the freshness status from the sensor module, and allowing the sensor module to detect activation of the RFID device; and an indicator communicatively coupled to the sensor module, the indicator being adapted to selectively activate and communicate the freshness status by use of a humanly perceivable signal under control of the sensor module; wherein the sensor module is adapted to selectively activate the indicator in response to detecting activation of the RFID device.

Although the systems for monitoring based on RFID technology as illustrated above comply with the purpose they are conceived for, they have some limitations, including the fact that the accuracy of the measurements related to the degree of ripeness of fruits is limited and, moreover, said systems are not suitable for continuous measurements on a plurality of fruits.

The main purpose of the present invention is thus to provide a system and a method based on RFID technology for monitoring the state or degree of ripeness of a plurality of fruits which allow for a continuous analysis of the fruits to be monitored over a preset time period and, at the same time, a higher accuracy and/or precision of the electromagnetic measurements related to the state or degree of ripeness of said fruits, so as to overcome the limitations of the prior art described above.

Another purpose of the present invention is to provide a system and a method for monitoring the state or degree of ripeness of a plurality of fruits as above which are effective and reliable in the detection of the actual state of the fruit and which can be made and used with low costs.

### Summary of the Invention

Said purposes are primarily achieved by a support for monitoring the degree of ripeness of a plurality of fruits, in particular tropical fruits, as defined in appended claim 1.

Particular embodiments of said support are disclosed in dependent claims 2 to 4.

The present invention also relates to a system for monitoring the degree of ripeness of a plurality of fruits, in particular tropical fruits, as defined in appended claim 5.

Particular embodiments of said system are disclosed in dependent claims 6 to 11.

In a further aspect, the present invention relates to a method for monitoring the degree of ripeness of a plurality of fruits as defined in appended claim 12.

Particular embodiments of said method are disclosed in dependent claim 13.

Finally, the present invention also relates to a stillage as defined in appended claim 14.

Particular embodiments of said stillage are disclosed in dependent claims 15 and 16.

Further features and advantages of the present invention will result from the following description of some preferred embodiments, said description being given by way of a non-limiting example with reference to the accompanying figures.

### Brief description of the figures

In the figures:
- Figure 1 shows a perspective view of a tray of a support according to an embodiment of the present invention;
- Figures 2-4 show respectively a top view and side views of the tray of the support of Figure 1;
- Figure 5 shows a perspective view of a base of a support according to an embodiment of the present invention, said base being suitable to be coupled to the tray of Figure 1;
- Figures 6-8 show respectively a top view and side cross-section views of the base of the support of Figure 1;
- Figures 9-10 show each a view of a detail of a support according to another embodiment of the present invention;
- Figure 11 shows a side view of a stillage according to an embodiment of the invention;
- Figure 12 shows a top view of the stillage of Figure 11;
- Figure 13 shows a cross-section view of the stillage of Figure 11 according to the line A-A of Figure 12;
- Figure 14 shows a cross-section view of the stillage of Figure 11 according to the line B-B of Figure 12;
- Figure 15 shows a schematic view of a detail of the system for monitoring the state or degree of ripeness of a plurality of fruits according to the invention;
- Figure 16 shows a schematic view of a detail of the monitoring system according to another embodiment of the invention.

### Detailed description

As mentioned previously, the monitoring system according to the invention comprises at least one RFID tag operating in the UHF band associated to each cavity for containing a fruit.

The RFID tag is by itself conventional and comprises a microchip and a RF antenna mounted on a substrate. In use, the substrate is used to attach the RFID tag directly on the fruit, or close to or in contact with it, in particular by fixing, for example with adhesives, the RFID tag in the cavity for containing said fruit.

The microchip has a printed circuit comprising an analog transponder for detecting and decoding a RF signal of interrogation and a decoder-processor for executing the RFID function and providing an analog-digital conversion for the communication with the transponder. The printed circuit further comprises a nonvolatile memory containing predetermined information which can be accessed using the decoder-processor.

Said predetermined information may be, for example, information related to the food (fruit) of application to be monitored, such as fruit type, origin, price, date of import and packaging.

The antenna of each RFID tag may be made of a flexible material, in particular of a metal material such as copper, aluminum or silver, and is operatively connected to the microchip. Advantageously, the RFID antenna may be made of a material suitable for the contact with food or it may be made suitable for the contact with food, for example by coating the material constituting the antenna with a film of a material suitable for food use, for example a film of a polymer material.

As known in the art, the printed circuit can recover the data stored by the memory and modulate an output RF signal by means of the transponder to transmit the data recovered to an interrogator, for example a reader. In addition to the recovery and the transmission of the data previously recorded in the memory, the RFID tag can also allow the registration of new data in the memory which overwrite the previous data or are additional to those data.

The RFID tag may also include other discrete electronic components such as capacitors, transistors, and diodes as well as logical elements for controlling the various components of the RFID tag.

The RFID tag may also include a battery or alternatively it may be of the passive type, in which case the antenna is used to provide power (to activate) the RFID circuit through an electromagnetic field provided by an interrogation device (reader) at the time of the interrogation of the RFID tag.

Within the scope of the present invention, the RFID tag is of the type operating in the UHF (Ultra High Frequency) band, thus with a circuit operating at a frequency preset in the UHF band. Preferably, the operative frequency is between 600 MHz and 1200 MHz.

The monitoring system according to the invention further comprises a device for interrogating comprising a reader and at least one interrogation antenna operatively associated to at least one RFID tag. In accordance with an aspect of the present invention, each interrogation antenna is arranged in a designated seat obtained in the support for the fruits and is located close to, preferably under, a respective cavity for containing a fruit or close to a group of containing cavities. Each interrogation antenna is capable of distributing a strong and substantially uniform electromagnetic field in a proximity detection area in such a way to interact with the RFID tags associated to one or more containing cavities and located in the vicinity.

Each interrogation antenna operates in the UHF band, as defined above, and is preferably an RFID near-field UHF antenna which is compact and has a thin shape, for example with the form of a loop or a printed patch.

In the present invention, each interrogation antenna is arranged inside the support in the designated housing seat and is operatively connected to an interrogation electronic component, such as a reader, located outside the support for fruits. In this regard, the support may be provided with connection means, such as one or more connection ports, to put in electrical communication each interrogation antenna with the reader, for example via designated connection cables which are connected to the opposite ends in respective ports of the reader and of an interrogation antenna, or through multiplexer connections wherein each multiplexer connection connects a plurality of interrogation antennas to the reader.

In the present invention, also the RF reader operates in the UHF band and can interrogate the RFID tags associated to the fruits through the interrogation antennas arranged in the support and recover the data from their memory by emitting a RF signal of interrogation compatible with the circuit of each RFID tag which is such to "activate" said circuit and to produce a response signal for each RFID tag through the transponder which is read and recorded by the reader.

The reader may be of the fixed type or advantageously of the portable type.

It has been surprisingly found that by using a system of measurement based on RFID technology with a fixed setup, that is to say wherein the RFID tags associated to the cavities for containing fruits and the interrogation antennas of the device for interrogating said RFID tags are maintained in a fixed position and at a short distance from each other, in particular close to each other, one achieves an improvement of accuracy and/or precision of the single measurements of electromagnetic signals, for example in frequency and/or power, reflected by the RFID tags and related to the state or degree of ripeness of the respective fruits.

Moreover, advantageously, by using a plurality of RFID tags associated to each containing cavity and therefore to each fruit, it is possible to obtain more electromagnetic signals of response of the RFID tags from each fruit and in different areas from the same, allowing to improve the precision of prediction of the state or degree of ripeness of each fruit.

As regards the method for monitoring the degree of ripeness of a plurality of fruits according to the invention, in a preliminary step the fruits to monitor are each arranged in a respective containing cavity of the support according to the invention and to each of the fruits is associated at least one RFID tag arranged in contact with or close to the respective fruit. In this regard, the RFID tags may be preemptively arranged in the respective containing cavities, for example attached on the bottom and/or on the side walls delimiting said cavity, thereby constituting part of the support according to the invention so that, in use, with the fruits arranged in the respective containing cavities the above-mentioned RFID tags are in contact with or close to the respective fruits, preferably in contact with said fruits. Alternatively, the RFID tags may be directly attached on the fruits whose state or degree of ripeness is to be monitored, in one or more areas of the same, for example a basal area and/or one or more side areas of the fruit, preferably equatorial areas. In the present invention, mixed arrangements comprising RFID tags attached to the fruits and RFID tags arranged in the respective cavities for containing said fruits are also considered.

Subsequently, an electromagnetic field (or signal) in the UHF band is generated by the reader which is irradiated by each interrogation antenna so as to run over one or more RFID tags operatively associated thereto, said RFID tag or tags being in turn associated to one or more containing cavities close to said interrogation antenna. Each electromagnetic field irradiated by an interrogation antenna involves then essentially the RFID tags of the containing cavities associated thereto. Said electromagnetic field or signal may have a preset frequency and/or phase and/or power or may be in the form of a scan within a preset range of frequency and/or phase and/or power.

From each RFID tag associated to a cavity for containing a fruit is therefore obtained a backscattered /reflected electromagnetic signal of response towards the respective antenna whose frequency and/or phase and/or power is affected by the peculiar dielectric characteristics of the fruit at issue that change over time in connection to the changes the fruit undergoes due to ripening (moisture, acidity, sugars) or to physiological alterations (texture) that occur during its storage. Consequently, each response signal deviates from the expected response signal obtainable in the absence of the perturbation of the dielectric environment around the RFID tag which is due to the presence of the fruit at issue.

Said deviation translates in an electromagnetic signal transmitted (i.e. reflected/backscattered) by each RFID tag to the interrogation antenna associated thereto, wherein each transmitted electromagnetic signal has a modified frequency and/or phase and/or power related to the degree of ripeness of a respective fruit or, in other words, in a variation of the frequency and/or phase and/or power (or radiation efficiency) with respect to the preset value, which variation is related to or can be related to the degree of ripeness of the fruit at issue on which said RFID tag is directly or indirectly applied.

The transmitted signals are recorded by the respective reading antennas and by the reader, and the data relative to or related to the frequency and/or phase and/or power of the transmitted signal and/or the data relative to or related to the variation of frequency and/or phase and/or power between the transmitted signal and a reference value of frequency and/or phase and/or power recorded by the reader are processed by the processing unit so as to produce a piece of information more readily "readable" and preferably suitable to be displayed which is related to the state or degree of ripeness of each fruit located in the containing cavities of the support. The reference values of frequency and/or phase and/or power may be set in the RFID tags or stored in a database in communication with the processing unit.

In this regard, it is possible to use classification/learning algorithms, supervised or non-supervised, which, starting from the transmitted data, distinguish the various levels of ripeness of the single fruits.

The processing may be implemented by suitable electronic means/components which use one or more of the above algorithms and are located directly in the fixed reader or in the portable reader. Alternatively, the data detected by the reader may be transmitted and processed by an external electronic device, for example a computer or a smartphone.

At the end of the processing, an information response about the state of ripeness of single fruits is obtained, which may be suitably displayed in a local or remote display device, for example a screen of any electronic device. In particular, it may be displayed using a screen of the same reader device.

The information may be in the form of any graphic composition (writing, graphics and/or images, etc.) that can be identified by the user, whether he/she is a logistics-responsible employee or a final consumer, as representative of the state of ripeness of the fruit.

In another embodiment, the information may be provided in the form of a QR code which is preferably dynamic and preferably shows all the relevant or desired information about the tracking and ripeness of the fruits and is capable of connecting to the web.

According to an embodiment of the present invention, the processed data relative to or related to the frequency and/or phase and/or power of the signals transmitted by said RFID tags may be compared, for example by a reader or an electronic device, with reference signal data relative to or related to an initial state before the ripening of the fruit at issue. Said reference data may be contained in a database for each fruit of interest or they may be assessed on the fruits to monitor, also using the system according to the invention, before the ripening of the same, for example right after the harvest or the positioning of said fruits in a ripening cell.

From this comparison, an information response for each fruit related to the variation between the processed data relative to said fruit and reference data is obtained, and said information response can be used to classify the single fruits according to their state or degree of ripeness and said classification of each fruit may be displayed by the display means.

In particular, the reference data may be classified, for each type of fruit, into classes of ripeness of the fruit at issue and the information response obtained after the comparison of the data relative to or related to the frequency and/or phase and/or level of power of the signal transmitted to said reader and processed by the processing means with the reference data may be the association and the display of the class they belong to which corresponds to each reading made by the RFID reader.

For example, the reference data may be classified, for each fruit, into three classes which respectively correspond to the situation wherein the fruit is not ripe yet, it is in suitable/optimal ripening conditions for consumption or has an altered texture such as to limit/compromise its consumption, and the information response corresponding to one of these situations and displayed by the display means can be that of a fruit belonging to the class "unripe", "ripe" or "very ripe", respectively. In an embodiment of the invention, each class of classification of the fruits can be subdivided in subclasses for a more functional classification of the fruit according to particular or specific needs. For example, the class "ripe" may comprise subclasses which are functional for the proper placement of the fruit in the distribution chain, such as "stock", "grocery" and "consumer".

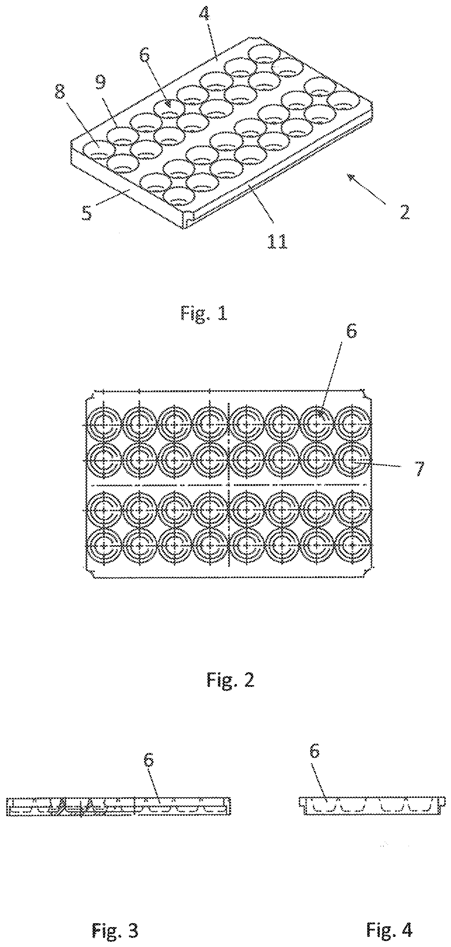

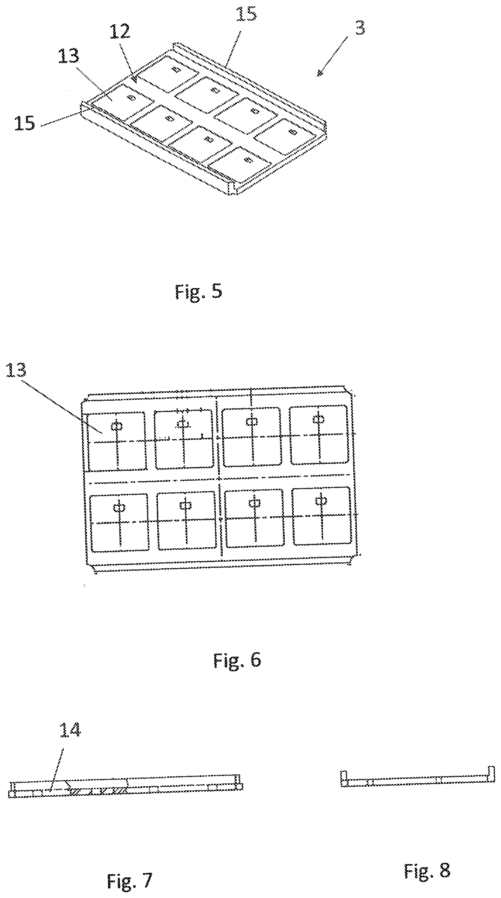

Figures 1-10 show a support for a plurality of fruits which is suitable for being used a system for monitoring the state or degree of ripeness of said fruits according to non-limiting embodiments of the present invention.

The support comprises a tray 2 and a base 3 that are intended to be removably coupled with each other with the tray 2 arranged on the base 3.

More in detail, the tray 3 has a substantially rectangular form and comprises an upper wall 4 and side walls 5 which extend downward from said upper wall 4. The tray 3 further comprises a plurality of cavities 6 suitable for containing a plurality of fruits, one for each fruit 10, which are preferably integrally formed with the material of the tray 3 and extend downward from said upper wall 4.

Each cavity 6 is delimited by a bottom wall 7 and at least one side wall 8 that ends in an upper edge 9 which defines the cavity opening 6. The cavities 6 have suitable sizes such as to preferably partially contain/house the fruits to monitor associated thereto, i.e. an upper portion of each fruit 10 protrudes at the top from a respective containing cavity 6 when arranged therein.

In the present embodiment, each cavity 6 has substantially the shape of a vase of small height and circular section with a diameter which is smaller on the bottom 7 and increases from the bottom 7 up to the opening 9. Obviously, the containing cavities 6 can also have different shapes, for example with square or rectangular section.

In the present embodiment, the containing csvities 6 are arranged one after the other along parallel rows extending in the longitudinal direction of the tray 2 and are substantially adjacent to each other in each of said rows.

In accordance with an aspect of the present invention, to each cavity for containing a fruit 10 is associated at least one RFID tag. Said at least one RFID tag may be arranged, for example, on the bottom 7 and/or on the side walls 8 of each containing cavity 6 and/or directly on the fruit 10 arranged in said containing cavity 6.

The tray 2 further comprises a pair of protrusions 11 on two opposite sides of the same, in this instance the long sides of the tray 2, which extend outwards from the upper wall 4 as an extension of the latter. The protrusions 11 extend substantially along the whole length of the respective opposite sides of the tray 2 and can be advantageously used as grip elements for the user for handling the tray 2.

The base 3 has a substantially rectangular shape comprising a lower portion 12 in which a plurality of housing seats 14 are obtained which are suitable for containing respective interrogation antennas 13. In particular, the interrogation antennas 13 are arranged one after the other in the respective housing seats 14 along parallel rows that extend in the longitudinal direction of the lower portion 12 of the base 3 so as to be located substantially under and close to one or more containing cavities 6 with the tray 2 arranged on the base 3 and coupled thereto.

In this way, each interrogation antenna 13 can be operatively associated to the RFID tags associated to one or more cavities 6 for containing respective fruits located close thereto, with said RFID tags being arranged in contact with or close to said fruits (for example arranged in the containing pits 6 and/or directly attached to the respective fruits).

For example, in the present embodiment, each interrogation antenna 13 can be operatively associated to the RFID tags associated to four adjacent containing cavities 6 arranged in opposite pairs on adjacent rows.

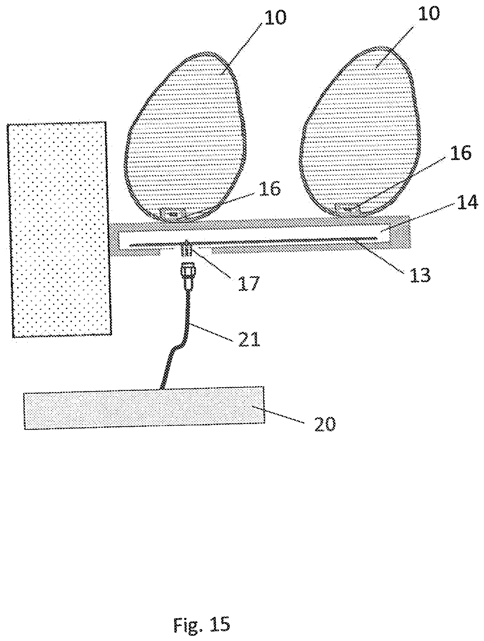

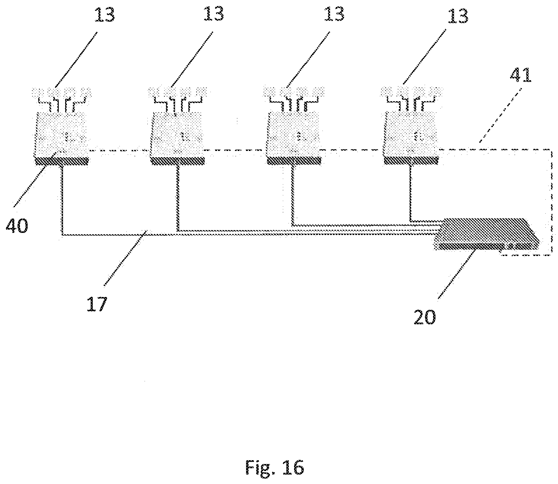

Moreover, the housing seats 14 containing the respective antennas 13 have each an aperture at a lower side of the lower portion of the base 3 at which connection means are arranged, for example a port 17. This allows to put each antenna 13 in electrical communication with a reader 20, for example via a cable 21, as schematically illustrated in Figure 15. Alternatively, the antennas 13 can be connected in groups to the reader 20 via a multiplexer 40 and moreover an ethernet connection cable 41 may be provided, as schematically illustrated in Figure 16.

In this way, each antenna 13 can irradiate an electromagnetic field (or signal) in the UHF band generated by the reader 20, that runs over one or more RFID tags operatively associated to the antenna and associated to one or more cavities for containing respective fruits, and can transmit the response signals received from said RFID tags and related to the state or degree of ripeness of said fruits toward the reader 20 for processing and subsequent displaying.

The base 3 further comprises a pair of protrusions 15 which extend upwards from the opposite lateral ends of the lower portion 12 of the base 3, in this instance from the ends of the long sides of the base 3.

In the present embodiment, the lower portion 12 of the base with the upper protrusions 15 has a substantially complementary shape to that of the tray 2, so as to allow to house said tray 2 in a seat delimited by the inner walls of the protrusions 14 and by the upper wall of the lower portion of the base 2 with the protrusions 11 which extend towards the outside of the tray 2 abutting on the upper part of the upper surface of the protrusions 15 oriented toward the top of the base 3.

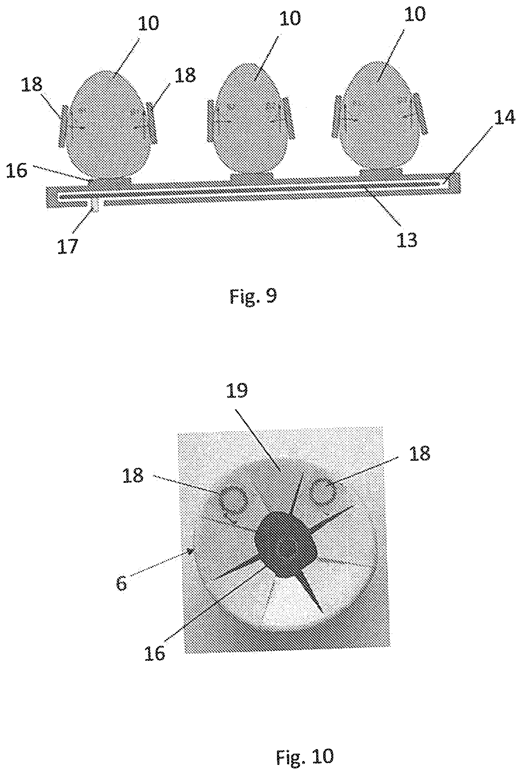

Figure 9 schematically shows an example of arrangement of RFID tags associated with respective containing cavities (the tags being not shown in Figure 9) of the support according to the invention.

In such example, with each containing cavity is associated a plurality of RFID tags comprising a first tag 16 arranged, in use, in contact with the base of a respective fruit 10, and second tags 18 arranged, in use, in contact with or close to a respective side area of said fruit 10, in this instance a central or equatorial area. In this case, the first tag 6 may be arranged (for example attached) on the bottom 7 of a containing cavity 6 while the second tags 18 may be arranged on the side walls 8 of the containing cavity 6, said RFID tags 16, 18 thereby constituting integral part of the support according to the invention. Alternatively, the RFID tags 16, 18 may be attached, in use, directly to the respective fruits, with the first tag 16 attached to the base of the fruit 10 and the second tags 18 attached at the side areas, for example in an equatorial area, of said fruit.

The second tags 18 may be arranged perpendicularly with respect to a transverse plane which crosses the fruit 10, for example in the equatorial area, or with a preset predefined angle with said plane, as illustrated for example in Figure 9.

Figure 10 schematically shows another example of arrangement of RFID tags associated with a containing cavity according to another embodiment of the support according to the invention.

In such example, each containing cavity 6 of the support further has, along the whole perimeter of the opening 8, a succession of tongues 19 folded inwards, i.e. that extend from the opening 8 of the containing cavity 6 toward the inside thereof. Said tongues advantageously facilitate the insertion of the fruit 10 in the respective containing cavity 6 thereby ensuring the latter to be centered and/or maintained in position by interference with said fruit 10.

Moreover, in this example, with each containing cavity 6 is associated a first RFID tag 16 arranged (for example attached) on the bottom 7 of the containing cavity 6 and second tags 18 arranged (for example attached) on respective tongues 19, said first RFID tag 16 and said second RFID tags coming, in use, in contact with a respective fruit 10 arranged in said containing cavity 6. This has the advantage to facilitate loading and unloading the fruits from the cavities 6.

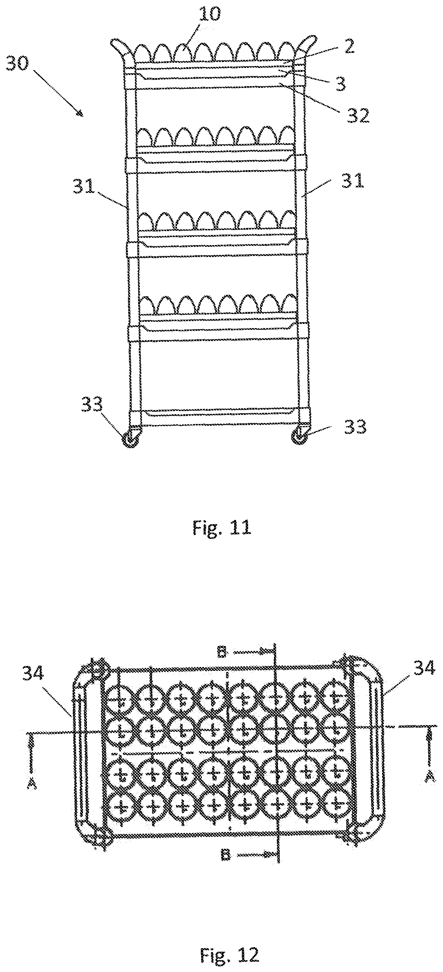

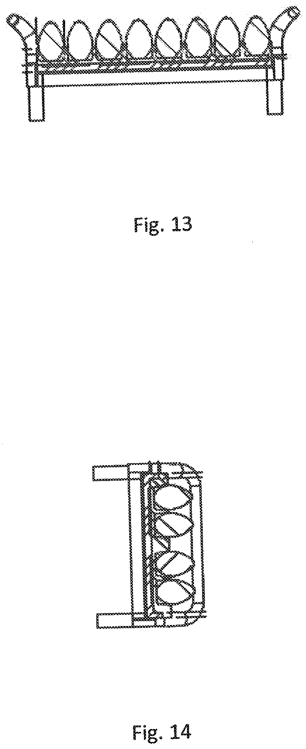

Figures 11-14 show a stillage according to an embodiment of the invention, wherein said stillage is referred to as a whole with reference number 30.

The stillage 30 comprises a frame with four tubular vertical supporting elements 31 and a plurality of shelves 32 arranged between the supporting elements 31 at predetermined heights between each other and fixed to said supporting elements 31 by suitable fixing means. In particular, in the present embodiment, the tubular elements 31 pass through respective holes (not shown) formed closed to the corners of the shelves 32 and are provided with conventional anchorage systems which insert between the holes of the shelves 32 so as to maintain them in position.

The shelves 32 have each a bearing seat adapted to receive a respective support comprising a tray 2 and a base 3 as previously described.

Moreover, the stillage 32 has four small wheels 33 fixed each to the lower end of a respective vertical supporting element 31 so that it is advantageously transportable for example by an operator.

The stillage has also a pair of opposite handles 34 fixed each to the upper ends of two opposite supporting elements 31.

A stillage as described above in association with respective supports according to the invention which contain fruits in the respective containing cavities can be advantageously used, for example, in a ripening chamber for monitoring the state or degree of ripeness of said fruits by constantly performing readings, over a preset time period, of the signals transmitted by the at least one RFID tag associated with each containing cavity (and therefore with each fruit), after activation, to the respective interrogation antenna and therefore to an external reader which processes said data and displays a piece of information related to the state of ripeness of each of said fruits.

In an embodiment, the stillage 30 comprises, integrated, a reader of the RFID tags associated to the containing cavities of the supports, said processing means (for example a computer) and said display means. This advantageously allows to transport the whole monitoring system in a simple way, thanks to the movable stillage, as needed.

In another embodiment, the reader 20 of the RFID tags associated to the containing cavities 6, the above-mentioned processing means and the above-mentioned display means are instead delocalized with respect to the stillage 30 and are operatively connected to each other and to the supports containing the cavities 6 for containing fruits during the use of the system according to the invention. For example, the stillage 30 with the supports containing the fruits to monitor can be placed in a fixed way in a chamber for ripening the fruits and the reader 20 of the RFID tags, the above-mentioned processing means and/or the above-mentioned display means may be arranged outside the ripening chamber and operatively connected, in use, to each other and to the supports containing the cavities for containing fruits.

In the light of the above, the RFID system according to the invention and the method for its use in monitoring the state or degree of ripeness of a plurality of fruits achieve the predetermined purposes and have several advantages.

In fact, thanks to the fact that the monitoring system according to the invention uses a fixed setup with interrogation antennas arranged in a fixed way close to the RDID tags associated therewith, the variability of the electromagnetic signal of response transmitted by the RFID tags to the reader is reduced and the signal-noise ratio (SNR) is improved, thereby obtaining a significant improvement of the accuracy and/or of the precision of the single measurements of electromagnetic signals, for example in frequency, phase and/or power, reflected by the RFID tags and related to the state or degree of ripeness of the respective fruits.

Moreover, advantageously, by using a plurality of RFID tags associated to each containing cavity and therefore to each fruit, it is possible to obtain more electromagnetic signals of response of the RFID tags from each fruit and in different areas from the same, allowing to improve the precision of prediction of the state or degree of ripeness of each fruit. In this regard, it is also possible to intercept the ripening of the fruits at an early stage, thereby anticipating the monitoring step.

Moreover, advantageously, the system according to the invention allows for continuous measurements or measurements at preset intervals over a preset time period on a plurality of fruits, for example of a production batch or over the whole process of fruit ripening in a ripening chamber, thereby obtaining a situation which is substantially in "real time" and representative of the state or degree of ripeness of all the above-mentioned fruits. It is also worth noting that the use of the monitoring system according to the invention does not require interruptions in the functioning of the process of ripening in chamber.

The monitoring system according to the invention is also effective and reliable and, at the same time, it can be produced at relatively low costs since RFID tags do not require special detection sensors and moreover no expensive analyzers and/or electronic components are required for its production. In any case, the RFID tags, if it is required, can also envisage the presence of sensors for specific needs, for example, for detecting other parameters related or not related to the fruit ripening, such as for example temperature, humidity, etc.

Moreover, the monitoring system according to the invention provides rapid readings of the degree of ripeness on a plurality of fruits, thereby being very convenient and practical in use. In this regard, it is also worth noting that the use of RFID tags integral with the containing cavities of the support makes the operations of loading and unloading the fruits from the respective cavities of the support rapider and more efficient.

In the light of the above, the system and the method for monitoring the degree of ripeness of a plurality of fruits according to the invention are very suitable for the use in the supply chain (storage, transport, over-the-counter sale, etc.) as well as in the scope of post-harvest monitoring and of monitoring the process of ripening in chamber with the purpose to preserve as long as possible the quality of fruit products, especially tropical fruits. In this way, waste is reduced and it is ensured that the fruits reach the market when they are at the proper degree of ripeness. In order to meet particular and specific needs, a skilled in the art can make several modifications and variants to the system and the method for monitoring the degree of ripeness of a plurality of fruits according to the invention.

For example, although the monitoring system of the invention has been described with reference to the detection of the degree of ripeness of fruits, it is evident that said system can be applied in the same way or in a similar way to any perishable food in so far as said food has peculiar dielectric characteristics that change over time in connection to the changes and/or physiological alterations that said food may undergo during its storage.

## Claims

1. A support for monitoring the state or degree of ripeness of a plurality of fruits, comprising
- a plurality of cavities (6) for containing respective fruits (10), one cavity per each fruit, wherein each containing cavity (6) has a bottom (7) and at least one side wall (8),
**characterized in that** said support further comprises
- a plurality of RFID tags (16, 18) associated to each cavity (6) for containing a fruit (10), said RFID tags being attached on said bottom (7) and/or on said at least one side wall (8) or being directly attached on said fruit (10),
and at least one housing seat (14), containing a respective interrogation antenna (13), each interrogation antenna (13) being, in use, operatively associated to said plurality of RFID tags (16, 18).

2. The support according to claim 1, wherein said plurality of tags comprise a first RFID tag (16) arranged, in use, in contact with a basal area of said fruit (10), and at least one second RFID tag (18) arranged, in use, in contact with a side area, preferably an equatorial area, of said fruit (10).

3. The support according to claim 1 or 2, wherein said at least one side wall (8) ends in an upper edge or opening (9) with perimeter extension and said containing cavity (6) has at least one tongue (19) extending from said upper edge or opening (9) toward the inside of said containing cavity (6), and a plurality of RFID tags (16, 18) associated to each cavity (6) for containing a fruit (10), said plurality of tags comprising a first RFID tag (16) applied on the bottom (7) of said containing cavity (6), and at least one second RFID tag (18) applied on said at least one side wall (8) and/or on said at least one tongue (19) of said containing cavity (6).

4. The support according to any one of claims 1 to 3, comprising a base (3) and a tray (2) removably associated or coupled with each other, the tray (2) comprising said cavities (6) for containing respective fruits (10), and the base (3) comprising at least one housing seat (14) and a respective interrogation antenna (13) housed in said seat (14), each housing seat (14) with the respective interrogation antenna (13) being arranged under a respective group of cavities (6) for containing fruits (10) and each interrogation antenna being, in use, operatively associated to the RFID tags (16, 18) associated to the containing cavities (6) of the respective group.

5. A system for monitoring the degree of ripeness of a plurality of fruits (10), in particular tropical fruits, comprising
- a support according to claim 1, in which said plurality of RFID tags (16, 18) associated to each cavity (6) for containing a fruit (10) operate in the UHF band),
- an interrogation device for interrogating said plurality of RFID tags, comprising a reader (20), which is in electrical communication with said at least one interrogation antenna (13), and
- means for processing electromagnetic signals or data relative to or related to the degree of ripeness of said fruits (10) coming from the reading of said RFID tags (16, 18) by said interrogation device.

6. The system according to claim 5, wherein said plurality of RFID tags (16, 18) associated to each cavity (6) for containing a fruit (10) comprise a first RFID tag (16) arranged, in use, in contact with a basal area of said fruit (10), and at least one second RFID tag (18) arranged, in use, in contact with a side area, preferably an equatorial area, of said fruit (10).

7. The system according to claim 5 or 6, wherein said at least one side wall (8) ends in an upper edge or opening (9) with perimeter extension and each containing cavity (6) has at least one tongue (19), preferably a plurality of tongues (19), extending from said upper edge or opening (9) toward the inside of said containing cavity (6).

8. The system according to claim 7, wherein each containing cavity (6) comprises a first RFID tag (16) applied on the bottom (7) of said containing cavity (6), and at least one second RFID tag (18) applied on said at least one side wall and/or on said at least one tongue (19) of said containing cavity.

9. The system according to any one of claims 5 to 8, wherein said support comprises a base (3) and a tray (3) which are removably associated or coupled with each other, the tray (3) comprising said cavities (6) for containing respective fruits (10), and the base (3) comprising at least one housing seat (14) and a respective interrogation antenna (13) housed in said seat (14), each housing seat (14) with the respective interrogation antenna (13) being arranged under a respective group of cavities (6) for containing fruits (10) and each interrogation antenna (13) being operatively associated to the RFID tags (16, 18) associated to the containing cavities of the respective group.

10. The system according to any one of claims 5 to 9, further comprising local or remote display means which are in communication with the processing means for displaying information about the degree or state of ripeness of fruits (10) related to the data processed by the processing means.

11. The system according to any one of claims 5 to 10, wherein said processing means are integrated in said reader (20).

12. A method for monitoring the degree of ripeness of a plurality of fruits (10) comprising the steps of:
- providing at least one support according to any one of claims 1 to 4;
- arranging a plurality of fruits (10) in respective containing cavities (6) of said at least one support with a plurality of RFID tags (16, 18) operating in the UHF band and associated to each containing cavity (6), wherein said RFID tags are attached on said bottom (7) and/or on said at least one side wall (8) or are directly attached on said fruit (10),
- generating at least one electromagnetic signal in the UHF band by means of the reader (20) of said interrogation device, each electromagnetic signal generated by said reader (20) being irradiated/directed by an interrogation antenna (13) so that it involves the RFID tags of at least one cavity (16) for containing a fruit which is/are operatively associated to said interrogation antenna (13),
- recording, by means of said interrogation device, a plurality of signals transmitted by the RFID tags (16, 18) associated to the respective cavities (6) for containing the fruits (10), each signal being related to the frequency and/or phase and/or power transmitted by the RFID tags (16, 18) associated to a respective cavity (6) for containing a fruit (10),
- processing data relative to or related to the frequency and/or phase and/or power of the transmitted signals and/or processing data relative to or related to the variation of frequency and/or phase and/or power between the signals transmitted by the RFID tags (16, 18) and a preset value of frequency and/or phase and/or power,
- displaying a piece of information about the degree or state of ripeness of said fruits (10) related to the processed data.

13. The method according to claim 12, further comprising:
- comparing the processed data relative to or related to the frequency and/or phase and/or power of the signals transmitted by said RFID tags (16, 18) with reference signal data relative to or related to an initial state before the ripening of said fruits (10), and
- classifying said fruits (10) according to their state or degree of ripeness on the basis of the variation between said processed data and said reference data,
- displaying a piece of information about the classification of said fruits.

14. A stillage (30) comprising a frame with vertical supporting elements (31), a plurality of shelves (32) arranged between said supporting elements (31) at predetermined heights between each other and connected to said supporting elements (31), and a plurality of supports as defined in any one of claims 1 to 4 arranged each on a respective shelf (32).

15. The stillage (30) according to claim 14, further comprising a plurality of small wheels (33) fixed at the lower end of said vertical supporting elements (31) and, optionally, at least one handle element (34), fixed at the upper ends of opposite supporting elements (31).

16. The stillage (30) according to claim 14 or 15, comprising, integrated, a reader (20) of the RFID tags (16, 18) associated to the containing cavities (6) of the supports, means for processing electromagnetic signals or data relative to or related to the degree of ripeness of fruits (10) coming from the reading of said RFID tags (16, 18) and said processing means and local or remote display means in communication with the processing means for displaying information about the degree or state of ripeness of fruits (10) related to the data processed by the processing means.

## Patentansprüche

1. Träger zur Überwachung des Reifezustands oder Reifegrads einer Vielzahl von Früchten, umfassend
- eine Vielzahl von Hohlräumen (6) zur Aufnahme jeweiliger Früchte (10), mit einem Hohlraum pro Frucht, wobei jeder Aufnahmehohlraum (6) einen Boden (7) und mindestens eine Seitenwand (8) aufweist,
**dadurch gekennzeichnet, dass** der Träger ferner umfasst
- eine Vielzahl von RFID-Tags (16, 18), die jedem Hohlraum (6) zur Aufnahme einer Frucht (10) zugeordnet sind, wobei die RFID-Tags an dem Boden (7) und/oder an der mindestens einen Seitenwand (8) angebracht oder unmittelbar an der Frucht (10) angebracht sind,
und mindestens einen Aufnahmesitz (14), der eine jeweilige Abfrageantenne (13) enthält, wobei jede Abfrageantenne (13), im Gebrauch, mit der Vielzahl von RFID-Tags (16, 18) operativ gekoppelt ist.

2. Träger nach Anspruch 1, wobei die Vielzahl von Tags einen ersten RFID-Tag (16), der, im Gebrauch, in Kontakt mit einem Grundbereich der Frucht (10) angeordnet ist, und mindestens einen zweiten RFID-Tag (18) umfasst, der, im Gebrauch, in Kontakt mit einem Seitenbereich, vorzugsweise einem Äquatorialbereich, der Frucht (10) angeordnet ist.

3. Träger nach Anspruch 1 oder 2, wobei die mindestens eine Seitenwand (8) in einem oberen Rand oder einer Öffnung (9) mit einer Umfangserstreckung endet und der Aufnahmehohlraum (6) mindestens eine Zunge (19), die sich von dem oberen Rand oder der Öffnung (9) in Richtung des Inneren des Aufnahmehohlraums (6) erstreckt, und eine Vielzahl von RFID-Tags (16, 18), die jedem Hohlraum (6) zur Aufnahme einer Frucht (10) zugeordnet sind, umfasst, wobei die Vielzahl von Tags einen ersten RFID-Tag (16), der auf den Boden (7) des Aufnahmehohlraums (6) aufgebracht ist, und mindestens einen zweiten RFID-Tag (18) umfasst, der auf die mindestens eine Seitenwand (8) und/oder auf die mindestens eine Zunge (19) des Aufnahmehohlraums (6) aufgebracht ist.

4. Träger nach einem der Ansprüche 1 bis 3, umfassend eine Basis (3) und eine Schale (2), die lösbar miteinander verbunden oder miteinander gekoppelt sind, wobei die Schale (2) die Hohlräume (6) zur Aufnahme jeweiliger Früchte (10) umfasst und die Basis (3) mindestens einen Aufnahmesitz (14) und eine jeweilige, in dem Sitz (14) untergebrachte Abfrageantenne (13) umfasst, wobei jeder Aufnahmesitz (14) mit der jeweiligen Abfrageantenne (13) unter einer jeweiligen Gruppe von Hohlräume (6) zur Aufnahme von Früchten (10) angeordnet ist und jede Abfrageantenne, im Gebrauch, mit den RFID-Tags (16, 18), die den Aufnahmehohlräumen (6) der jeweiligen Gruppe zugeordnet sind, operativ gekoppelt ist.

5. System zur Überwachung des Reifegrads einer Vielzahl von Früchten (10), insbesondere tropischer Früchte, umfassend
- einen Träger nach Anspruch 1, wobei die Vielzahl von RFID-Tags (16, 18), die jedem Hohlraum (6) zur Aufnahme einer Frucht (10) zugeordnet sind, im UHF-Band arbeitet,
- eine Abfrageeinrichtung zum Abfragen der Vielzahl von RFID-Tags, welche ein Lesegerät (20) umfasst, das mit der mindestens einen Abfrageantenne (13) elektrisch verbunden ist, und
- Mittel zum Verarbeiten elektromagnetischer Signale oder von Daten, die sich auf den Reifegrad der Früchte (10) beziehen oder damit in Zusammenhang stehen und aus dem Auslesen der RFID-Tags (16, 18) durch die Abfrageeinrichtung stammen.

6. System nach Anspruch 5, wobei die Vielzahl von RFID-Tags (16, 18), die jedem Hohlraum (6) zur Aufnahme einer Frucht (10) zugeordnet sind, einen ersten RFID-Tag (16), der im Gebrauch in Kontakt mit einem basalen Bereich der Frucht (10) angeordnet ist, und mindestens einen zweiten RFID-Tag (18) umfasst, der im Gebrauch in Kontakt mit einem Seitenbereich, vorzugsweise einem Äquatorialbereich, der Frucht (10) angeordnet ist.

7. System nach Anspruch 5 oder 6, wobei die mindestens eine Seitenwand (8) in einem oberen Rand oder einer Öffnung (9) mit einer Umfangserstreckung endet und jeder Aufnahmehohlraum (6) mindestens eine Zunge (19), vorzugsweise eine Vielzahl von Zungen (19), aufweist, die sich von dem oberen Rand oder der Öffnung (9) in Richtung des Inneren des Aufnahmehohlraums (6) erstrecken.

8. System nach Anspruch 7, wobei jeder Aufnahmehohlraum (6) einen ersten RFID-Tag (16), der auf den Boden (7) des Aufnahmehohlraums (6) aufgebracht ist, und mindestens einen zweiten RFID-Tag (18) umfasst, der auf die mindestens eine Seitenwand und/oder auf die mindestens eine Zunge (19) des Aufnahmehohlraums angebracht ist.

9. System nach einem der Ansprüche 5 bis 8, wobei der Träger eine Basis (3) und eine Schale (3) umfasst, die lösbar miteinander verbunden oder miteinander gekoppelt sind, wobei die Schale (3) die Hohlräume (6) zur Aufnahme jeweiliger Früchte (10) umfasst und die Basis (3) mindestens einen Aufnahmesitz (14) und eine jeweilige, in dem Sitz (14) untergebrachte Abfrageantenne (13) umfasst, wobei jeder Aufnahmesitz (14) mit der jeweiligen Abfrageantenne (13) unter einer jeweiligen Gruppe von Hohlräumen (6) zur Aufnahme von Früchten (10) angeordnet ist und jede Abfrageantenne (13) mit den RFID-Tags (16, 18), die den Aufnahmehohlräumen der jeweiligen Gruppe zugeordnet sind, operativ gekoppelt ist.

10. System nach einem der Ansprüche 5 bis 9, ferner umfassend lokale oder entfernte Anzeigemittel, die mit den Verarbeitungsmitteln in Kommunikation stehen, um Informationen über den Reifegrad oder den Reifezustand von Früchten (10), die sich auf die von den Verarbeitungsmitteln verarbeiteten Daten beziehen, anzuzeigen.

11. System nach einem der Ansprüche 5 bis 10, wobei die Verarbeitungsmittel in das Lesegerät (20) integriert sind.

12. Verfahren zur Überwachung des Reifegrads einer Vielzahl von Früchten (10), welches folgende Schritte umfasst:
- Bereitstellen mindestens eines Trägers nach einem der Ansprüche 1 bis 4;
- Anordnen einer Vielzahl von Früchten (10) in jeweilige Aufnahmehohlräume (6) des mindestens einen Trägers mit einer Vielzahl von im UHF-Band arbeitenden RFID-Tags (16, 18), die jedem Aufnahmehohlraum (6) zugeordnet sind, wobei die RFID-Tags an dem Boden (7) und/oder an der mindestens einen Seitenwand (8) angebracht oder unmittelbar an der Frucht (10) angebracht sind,
- Erzeugen mindestens eines elektromagnetischen Signals im UHF-Band mittels des Lesegeräts (20) der Abfrageeinrichtung, wobei jedes von dem Lesegerät (20) erzeugte elektromagnetische Signal durch eine Abfrageantenne (13) abgestrahlt/gerichtet wird, so dass es die RFID-Tags mindestens eines Hohlraums (16) zur Aufnahme einer Frucht einbindet, der/die mit der Abfrageantenne (13) operativ gekoppelt ist/sind,
- Aufzeichnen, mittels der Abfrageeinrichtung, einer Vielzahl von Signalen, die von den RFID-Tags (16, 18) übertragen werden, die den jeweiligen Hohlräumen (6) zur Aufnahme der Früchte (10) zugeordnet sind, wobei jedes Signal sich auf die Frequenz und/oder Phase und/oder Leistung bezieht, die von den RFID-Tags (16, 18), die einem jeweiligen Hohlraum (6) zur Aufnahme einer Frucht (10) zugeordnet sind, übertragen wird,
- Verarbeiten von Daten, die sich auf die Frequenz und/oder Phase und/oder Leistung der übertragenen Signale beziehen oder damit in Zusammenhang stehen, und/oder Verarbeiten von Daten, die sich auf die Änderung der Frequenz und/oder Phase und/oder Leistung zwischen den von den RFID-Tags (16, 18) übertragenen Signalen und einem voreingestellten Wert der Frequenz und/oder Phase und/oder Leistung beziehen oder damit in Zusammenhang stehen,
- Anzeigen einer Information über den Reifegrad oder Reifezustand der Früchte (10), die sich auf die verarbeiteten Daten bezieht.

13. Verfahren nach Anspruch 12, wobei das Verfahren weiter umfasst:
- Vergleichen der verarbeiteten Daten, die sich auf die Frequenz und/oder Phase und/oder Leistung der von den RFID-Tags (16, 18) übertragenen Signale beziehen oder damit in Zusammenhang stehen, mit Referenzsignaldaten, die sich auf einen Anfangszustand vor dem Reifen der Früchte (10) beziehen oder damit in Zusammenhang stehen, und
- Klassifizieren der Früchte (10) nach ihrem Reifezustand oder Reifegrad basierend auf der Variation zwischen den verarbeiteten Daten und den Referenzdaten,
- Anzeigen einer Information über die Klassifizierung der Früchte.

14. Palette (30), welche einen Rahmen mit vertikalen Stützelementen (31), eine Vielzahl von Ablagen (32), die zwischen den Stützelementen (31) mit vorbestimmten Höhen zwischen einander angeordnet und mit den Stützelementen (31) verbunden sind, und eine Vielzahl von Trägern nach einem der Ansprüche 1 bis 4 umfasst, die jeweils auf einer jeweiligen Ablage (32) angeordnet sind.

15. Palette (30) nach Anspruch 14, welche weiter eine Vielzahl kleiner Räder (33), die am unteren Ende der vertikalen Stützelemente (31) befestigt sind, und, optional, mindestens ein Griffelement (34) umfasst, das an den oberen Enden gegenüberliegender Stützelemente (31) befestigt ist.

16. Palette (30) nach Anspruch 14 oder 15, umfassend, als integrierte Elemente, ein Lesegerät (20) der RFID-Tags (16, 18), die den Aufnahmehohlräumen (6) der Träger zugeordnet sind, Mittel zum Verarbeiten elektromagnetischer Signale oder von Daten, die sich auf den Reifegrad von Früchten (10) beziehen oder damit in Zusammenhang stehen und aus dem Auslesen der RFID-Tags (16, 18) stammen, und die Verarbeitungsmittel sowie lokale oder entfernte Anzeigemittel, die mit den Verarbeitungsmitteln in Kommunikation stehen, um Informationen über den Reifegrad oder Reifezustand von Früchten (10), die sich auf die von den Verarbeitungsmitteln verarbeiteten Daten beziehen, anzuzeigen.

## Revendications

1. Support pour surveiller l'état ou le degré de maturité d'une pluralité de fruits, comprenant :
- une pluralité de cavités (6) pour contenir des fruits (10) respectifs, une cavité pour chaque fruit, dans lequel chaque cavité (6) comporte un fond (7) et au moins une paroi latérale (8),
**caractérisé en ce que** ledit support comprend en outre :
- une pluralité d'étiquettes RFID (16, 18) associées à chaque cavité (6) pour contenir un fruit (10), lesdites étiquettes RFID étant fixées sur ledit fond (7) et/ou sur ladite au moins une paroi latérale (8), ou étant directement fixées audit fruit (10),
et au moins un logement (14) recevant une antenne d'interrogation (13) respective, chaque antenne d'interrogation (13) étant, en utilisation, associée fonctionnellement à ladite pluralité d'étiquettes RFID (16, 18).

2. Support selon la revendication 1, dans lequel ladite pluralité d'étiquettes comprend une première étiquette RFID (16) disposée, en utilisation, au contact d'une zone basale dudit fruit (10), et au moins une seconde étiquette RFID (18) disposée, en utilisation, au contact d'une zone latérale, de préférence équatoriale, dudit fruit (10).

3. Support selon la revendication 1 ou 2, dans lequel ladite au moins une paroi latérale (8) se termine par un bord supérieur ou une ouverture (9) s'étendant sur son périmètre, et dans lequel ladite cavité (6) comporte au moins une languette (19) s'étendant depuis ledit bord supérieur ou ouverture (9) vers l'intérieur de ladite cavité (6), une pluralité d'étiquettes RFID (16, 18) étant associée à chaque cavité (6) pour contenir un fruit (10), ladite pluralité d'étiquettes RFID comprenant une première étiquette RFID (16) appliquée sur le fond (7) de ladite cavité (6), et au moins une seconde étiquette RFID (18) appliquée sur ladite au moins une paroi latérale (8) et/ou sur ladite au moins une languette (19) de ladite cavité (6).

4. Support selon l'une quelconque des revendications 1 à 3, comprenant une base (3) et un plateau (2) associés ou couplés de manière amovible, le plateau (2) comprenant lesdites cavités (6) pour contenir des fruits (10) respectifs, et la base (3) comprenant au moins un logement (14) et une antenne d'interrogation (13) respective reçue dans ledit logement (14), chaque logement (14) avec son antenne d'interrogation (13) respective étant disposé sous un groupe respectif de cavités (6) pour contenir des fruits (10), chaque antenne d'interrogation étant, en utilisation, associée fonctionnellement aux étiquettes RFID (16, 18) associées aux cavités (6) du groupe correspondant.

5. Système pour surveiller le degré de maturité d'une pluralité de fruits (10), notamment de fruits tropicaux, comprenant :
- un support selon la revendication 1, dans lequel la pluralité d'étiquettes RFID (16, 18) associées à chaque cavité (6) fonctionne dans la bande UHF ;
- un dispositif d'interrogation pour interroger lesdites étiquettes RFID, comprenant un lecteur (20), qui est en communication électrique avec ladite au moins une antenne d'interrogation (13) ; et
- des moyens pour traiter de signaux électromagnétiques ou des données relatifs ou liés au degré de maturité desdits fruits (10), provenant de la lecture desdites étiquettes RFID (16, 18) par ledit dispositif d'interrogation.

6. Système selon la revendication 5, dans lequel ladite pluralité d'étiquettes RFID (16, 18) associée à chaque cavité (6) comprend une première étiquette RFID (16) disposée, en utilisation, au contact d'une zone basale du fruit (10) et au moins une seconde étiquette RFID (18) disposée, en utilisation, au contact d'une zone latérale, de préférence équatoriale, dudit fruit (10).

7. Système selon la revendication 5 ou 6, dans lequel ladite au moins une paroi latérale (8) se termine par un bord supérieur ou une ouverture (9) et chaque cavité (6) comprend au moins une languette (19), de préférence une pluralité de languettes (19), s'étendant depuis le bord supérieur ou l'ouverture (9) vers l'intérieur de la cavité (6).

8. Système selon la revendication 7, dans lequel chaque cavité (6) comprend une première étiquette RFID (16) appliquée sur le fond (7) de ladite cavité (6) et au moins une seconde étiquette RFID (18) appliquée sur ladite au moins une paroi latérale et/ou sur ladite au moins une languette (19) de ladite cavité.

9. Système selon l'une quelconque des revendications 5 à 8, dans lequel le support comprend une base (3) et un plateau (3) associés ou couplés de manière amovible, le plateau comprenant les cavités (6) pour contenir des fruits (10) respectifs, et la base (3) comprenant au moins un logement (14) et une antenne d'interrogation (13) respective reçue dans ledit logement (14), chaque logement (14) avec son antenne d'interrogation respective (13) étant disposé sous un groupe respectif de cavités (6) pour contenir des fruits (10) et chaque antenne d'interrogation étant associée fonctionnellement aux étiquettes RFID (16, 18) associées aux cavités du groupe correspondant.

10. Système selon l'une quelconque des revendications 5 à 9, comprenant en outre des moyens d'affichage locaux ou distants en communication avec les moyens de traitement pour afficher des informations sur le degré ou l'état de maturité des fruits (10) correspondant aux données traitées par les moyens de traitement.

11. Système selon l'une quelconque des revendications 5 à 10, dans lequel les moyens de traitement sont intégrés audit lecteur (20).

12. Procédé de surveillance du degré de maturité d'une pluralité de fruits (10), comprenant les étapes de :
- fournir au moins un support selon l'une quelconque des revendications 1 à 4 ;
- disposer une pluralité de fruits (10) dans les cavités (6) respectives avec une pluralité d'étiquettes RFID (16, 18) fonctionnant dans la bande UHF et associées à chaque cavité (6), dans lequel lesdites étiquettes RFID sont fixées sur ledit fond (7) et/ou sur ladite au moins une paroi latérale (8), ou sont directement fixées audit fruit (10) ;
- générer au moins un signal électromagnétique dans la bande UHF au moyen du lecteur (20) du dispositif d'interrogation, chaque signal électromagnétique généré par ledit lecteur (20) étant rayonné/dirigé par une antenne d'interrogation (13) de manière à impliquer les étiquettes RFID d'au moins une cavité (16) pour contenir un fruit, laquelle ou lesquelles sont associées fonctionnellement à ladite antenne d'interrogation (13) ;
- enregistrer, au moyen dudit dispositif d'interrogation, une pluralité de signaux transmis par les étiquettes RFID (16, 18) associées aux cavités (6) respectives pour contenir les fruits (10), chaque signal étant lié à la fréquence et/ou à la phase et/ou à la puissance transmises par les étiquettes RFID (16, 18) associées à une cavité (6) respective pour contenir un fruit (10) ;
- traiter des données relatives ou liées à la fréquence et/ou à la phase et/ou à la puissance des signaux transmis et/ou traiter des données relatives ou liées à la variation de fréquence et/ou de phase et/ou de puissance entre les signaux transmis par les étiquettes RFID (16, 18) et une valeur prédéfinie de fréquence et/ou de phase et/ou de puissance ;
- afficher une information concernant le degré ou l'état de maturité desdits fruits (10) correspondant aux données traitées.

13. Procédé selon la revendication 12, comprenant en outre :
- comparer les données traitées relatives ou liées à la fréquence et/ou à la phase et/ou à la puissance des signaux transmis par lesdites étiquettes RFID (16, 18) avec des données de signaux de référence relatives ou liées à un état initial avant la maturation desdits fruits (10) ; et
- classifier lesdits fruits (10) selon leur état ou leur degré de maturité sur la base de la variation entre lesdites données traitées et lesdites données de référence ;
- afficher une information concernant la classification desdits fruits.

14. Structure porteuse (30) comprenant un bâti comportant des éléments porteurs verticaux (31), une pluralité d'étagères (32) disposées entre lesdits éléments porteurs (31) à des hauteurs prédéterminées les unes par rapport aux autres et reliées auxdits éléments porteurs (31), et une pluralité de supports tels que définis dans l'une quelconque des revendications 1 à 4, qui sont chacun disposés sur une étagère (32) respective.

15. Structure porteuse (30) selon la revendication 14, comprenant en outre une pluralité de roulettes (33) fixées à l'extrémité inférieure desdits éléments porteurs verticaux (31), et, optionnellement, au moins un élément de poignée (34) fixé aux extrémités supérieures d'éléments porteurs (31) opposés.

16. Structure porteuse (30) selon la revendication 14 ou 15, comprenant, de manière intégrée un lecteur (20) des étiquettes RFID (16, 18) associées aux cavités (6) des supports, des moyens de traitement de signaux électromagnétiques ou de données relatives ou liées au degré de maturité des fruits (10) provenant de la lecture desdites étiquettes RFID (16, 18), et lesdits moyens de traitement ainsi que des moyens d'affichage locaux ou distants en communication avec les moyens de traitement afin d'afficher des informations concernant le degré ou l'état de maturité des fruits (10), lesdites informations correspondant aux données traitées par les moyens de traitement.
